# EUROPÄISCHE PATENTSCHRIFT

(11) **EP 2 841 125 B1**
(45) Veröffentlichungstag und Bekanntmachung des Hinweises auf die Patenterteilung: **15.03.2017**
(21) Anmeldenummer: 13718506.2
(22) Anmeldetag: 18.04.2013
(51) Int. Cl.: A61M 1/16, A61J 1/14, B65D 81/00

(54) **BEUTEL MIT EINGESCHWEISSTEM BIEGESTEIFEN KUNSTSTOFFTEIL**
BAG WITH SHRINK-WRAPPED, FLEXURALLY RIGID PLASTIC PART
POCHE MUNIE D'UNE PIÈCE EN PLASTIQUE SOUDÉE RIGIDE EN FLEXION

(30) Priorität: 23.04.2012 DE 102012007904; 23.04.2012 US 201261636771 P
(43) Veröffentlichungstag der Anmeldung: 04.03.2015
(73) Patentinhaber: Fresenius Medical Care Deutschland GmbH, 61352 Bad Homburg (DE)
(72) Erfinder: KUGELMANN, Franz, 66606 St. Wendel/Bliesen (DE); HÖRMANN, Jörn, 66265 Heusweiler (DE)
(74) Vertreter: Herrmann, Uwe
(86) Internationale Anmeldenummer: PCT/EP2013/001155
(87) Internationale Veröffentlichungsnummer: WO 2013/159883

(56) Entgegenhaltungen:
- WO-A1-2011/070329
- WO-A2-2007/144427
- WO-A2-2009/052501
- US-A- 4 869 398
- US-A1- 2003 080 126
- US-A1- 2010 315 231

## Beschreibung

Die Erfindung betrifft das Gebiet der Bereitstellung von medizinischen Lösungen, insbesondere Lösungen für die Infusion und die Nierenersatztherapie. Gegenstand der Erfindung ist ein Beutel, insbesondere ein Mehrkammerbeutel für die Aufnahme von Konzentraten mit einer umfänglichen Abdichtung und einem Aufnahmebereich aus einem biegesteifen Material, der Zugabe- und/oder Entnahmeports für Flüssigkeiten umfasst.

Dialyseflüssigkeiten für die Hämodialyse oder Peritonealdialyse, sowie Flüssigkeiten und Austauschflüssigkeiten für Hämofiltrationsverfahren enthalten typischerweise gelöste Stoffe wie zum Beispiel:
- Elektrolyte Na, K, Mg, Ca, um einen annehmbaren Elektrolythaushalt des Patienten aufrecht zu erhalten
- Puffer (zum Beispiel Bikarbonat, Acetat, Laktat, usw.)
- Glukose (oder andere osmotische Agenzien), als osmotisches Mittel in der Peritonealdialyse oder zur Aufrechterhaltung des Blutzuckspiegels während der Hämodialyse und der Hämofiltration
- Säuren oder Salze von Säuren (zum Beispiel HCl bzw. Cl⁻, Essigsäure bzw. Acetat, Zitronensäure bzw. Citrat), die eventuell zur Neutralisation basischer Teil-Dialyselösungen beitragen oder als Gegenionen im elektrochemischen Gleichgewicht vorliegen.

Die für Dialyselösung verwendeten Substanzen sind im Allgemeinen nicht in einer gebrauchsfertigen Mischung lagerfähig, da die Substanzen eine gegenseitige Degradation verursachen können. Die geforderte Lagerstabilität einer Komponente kann Lagerbedingungen voraussetzen, die für andere Komponenten zur Degradation führt. Zum Beispiel ist Glukose, abhängig von der Konzentration in Lösung, nur in einem bestimmten sauren pH-Wert Bereich über längere Zeit lagerfähig, ohne dabei im übermäßigen Maß unerwünschten Degradationsvorgängen zu unterliegen. Gleichzeitig ist die in Dialyselösung häufig als Puffer verwendete Verbindung Natriumbikarbonat nicht unter solchen sauren Bedingungen lagerfähig, da Bikarbonat, abhängig vom pH-Wert, zur Zersetzung neigt und CO₂ freisetzen kann. Unter Zersetzungsbedingungen ändert sich die Konzentration von Bikarbonat, was aus therapeutischer Sicht inakzeptabel ist. Der steigende CO₂ Partialdruck stellt zudem Anforderung an die medizinischen Dialysegeräte, die zu technischen Problemen führt.

Es sind eine Vielzahl von alternativen Zusammensetzungen, Lagerbedingungen und Darreichungsformen von Dialyselösungen oder Konzentraten bekannt, die eine lange Lagerung ermöglichen. Bekannt ist die Aufteilung der Lösungskomponenten in eine Kombination von Teillösungen oder Konzentraten, so dass nur verträgliche Komponenten einer Teillösung oder eines Teilkonzentrats zusammen gelagert werden. Für Lösungen der Peritoneal Dialyse wird gängigerweise eine erste Teillösung, umfassend Glukose, das die Funktion des Osmotikums übernimmt, bei einem sauren pH Wert mit weiteren Elektrolyten, zum Beispiel Natrium, Kalzium, Magnesium, gelagert. Eine weitere basische oder gepufferte Teillösung ist notwendig, um mit der ersten sauren Teillösung eine physiologische oder wenigstens für die Therapie gebrauchsfertige Mischlösung aus dem ersten Teil und dem zweiten Teil zu liefern. Der zweite Teil besteht dabei häufig aus einer Lösung oder einem Konzentrat von Natriumbikarbonat und Natriumchlorid. Die Teillösungen oder Konzentrate werden dabei in mehreren Behältern oder in mehreren Kammern eines Behälters vorgelegt. Die Teillösungen oder Teilkonzentrate liegen getrennt vor, so dass keine gegenseitige Beeinflussung entsteht. Unmittelbar vor Gebrauch der Dialyselösung werden die getrennten Teillösungen oder Teilkonzentrate eventuell unter Zugabe weiterer wässriger Komponenten gemischt und für die Behandlung bereitgestellt.

In der Hämodialyse werden die Teillösungen oder Teilkonzentrate oftmals in der Dialysemaschine vor und während des Verlaufs der Behandlung gemischt und zu einer fertigen Dialyselösung hergestellt. Hierfür werden oft Teilkonzentrate in fester oder in flüssiger Form verwendet, die in einzelnen Behältern vorliegen und durch einen Anschluss an die Dialysemaschine mithilfe eines vorbereiteten Hydrauliksystems verdünnt, gemischt und zur fertigen verwendbaren Dialyselösung aufbereitet werden.

Andere Entwicklungen in der Dialyse gehen dazu über, die notwendigen Konzentrate in einem einzigen Behälter vorzulegen. Zum einen vereinfacht dies die Herstellung und Handhabung der Behälter, zum anderen wird dadurch auch das hydraulische System der Dialysemaschine vereinfacht, da mitunter nur eine Aufnahmeeinheit für die Teillösungen oder Konzentrate vorzusehen ist und weniger Anschlüsse notwendig sind, um die Lösung durch das hydraulische System aufzubereiten. Dieser Trend kann vor allem in der Akutdialyse beobachtet werden, da hier eine größere Mobilität der Behandlungssysteme gefragt ist.

In einer weiteren Variante werden Dialyselösungen für die Hämodialyse nicht während des Verlaufs der Behandlung aus Konzentraten hergestellt, sondern das gesamte benötigte Volumen der Dialyselösung wird in einem Batch in einem der Behandlung vorgelagerten Schritt aufbereitet. Das Batch wird in einem Tank bevorratet, der dafür vorbereitet ist, mit einer Dialysemaschine verbunden zu werden. In manchen Fällen ist der Tank ein integraler Bestandteil einer Dialysebehandlungseinheit oder kann in bestimmten Fällen auch wieder von diesem separat bewegt werden. Die Batchdialyse kann somit den Vorteil aufweisen, durch eine einmalige Zubereitung des Batches den Behandlungsplatz relativ ortsunabhängig zu wählen. Somit sind Behandlungsstationen an verschiedenen Orten einsetzbar, ohne auf eine Zubereitungseinheit von Dialyselösung oder einem Wasseranschluss, der das notwendige Wasser zur Verdünnung der Konzentrate liefert, angewiesen zu sein. In diesen Fällen wird die Dialyselösung an einer dafür vorgesehenen Einrichtung aus Konzentraten zusammengemischt und in einem meist mobilen Tank bevorratet.

Großvolumige Flüssigkeitsbeutel, worunter im Folgenden Beutel mit einem Fassungsvermögen ab 5 Litern zu verstehen sind, sind vor besondere mechanische Erfordernisse gestellt. Das Folienmaterial der Beutel muss eine genügend starke Schlagzähigkeit aufweisen, so dass beim Herunterfallen die Beutel nicht aufplatzen. Insbesondere sind diese Anforderungen bei niedrigen Temperaturen gefordert. Entsprechende Eignungstests bewerten die Festigkeit von Lösungsbeuteln durch einen Falltest. Bei diesem Test werden zuvor auf 4°C temperierte Beutel in einem entsprechend temperierten Raum aus einer Höhe von 2 Metern zu Boden fallen gelassen und das eventuell auftretende Berstverhalten der Beutel beurteilt. Darüber hinaus müssen die Schweißnähte der aus Folienabschnitten zusammengesetzten Beutel bestimmten Druckbeanspruchungen standhalten können, ohne dass es zu Undichtigkeiten des Beutels kommt. Die Beutelmaterialien müssen auch weiteren Anforderungen der Hitzesterilisierbarkeit und der Transparenz entsprechen. Insbesondere sind diese Erfordernisse für Lösungsbeutel relevant, die in sterilisierter und gebrauchsfertiger Form in den Handel kommen und vertrieben werden. Besonders die mechanischen Beanspruchungen, denen solche Beutel auf den Vertriebswegen ausgesetzt sind, führen zu Reklamationen und unangenehmen Nachforderungen der Kunden an den Hersteller. Derzeit werden Lösungsbeutel für die Peritonealdialyse, die Hämodialyse und der Hämofiltration gängigerweise in Größen bis zu 5 Litern vertrieben.

Lösungsbeutel mit einem größeren Fassungsvolumen sind aufgrund der Transportlogistik nur noch erschwert in gebrauchsfertiger Form auslieferbar. Für Beutel, die wie oben beschrieben in der Batchdialyse zur Anwendung kommen, ist die Herstellung der Lösungen am Behandlungs- und Gebrauchsort vorzusehen. Die Beutel müssen so vor Ort durch eine entsprechende Flüssigkeitsaufbereitungsanlage befüllt werden und sind innerhalb der Klinikstation, in gewissem Maße auch regional darüber hinaus, einsetzbar. Solche Batchsysteme können Beutelvolumina von 30 bis 120 Litern erfordern. Derart großvolumige Beutel weisen bereits durch ihr Eigengewicht eine starke Belastung auf geschweißte Abschnitte, z.B. Schweißnähte oder der eingeschweißten Ports auf.

Um den Beutel über einen Port befüllen oder über einen Port entleeren zu können, müssen diese Ports in die umfängliche Schweißnaht, die die Folienwandabschnitte aneinander fixiert und ein inneres Volumen des Beutels definiert, eingearbeitet werden. Die Ports weisen eine Durchgangsöffnung auf, die das Innere des Beutels mit dem äußeren Bereich verbindet und von Flüssigkeit durchströmbar ist. Das äußere Ende des Ports wird dabei mit weiteren Leitungen oder Anschlüssen des Beutelsystems oder der Behandlungsstation verbunden. Bekannt ist es dabei, den Einschweißbereich der Ports besonders für die Schweißverbindung mit dem Folienmaterial auszugestalten. Daher wurden sogenannte Schweißrippen entwickelt, die in Querrichtung zur Erstreckungsrichtung der Durchgangsöffnung der Ports und parallel zur Schweißnaht der Folie ausgerichtet sind. Schweißwerkzeuge pressen im Herstellprozess die Folien an diese relativ dünnen Schweißrippen, womit sich lokal ein sehr hoher Anpressdruck ergibt, der die Schweißnaht abdichtet.

Über die einfache Abdichtung hinaus müssen diese Verbindungen aber auch eine feste mechanische Verbindung zwischen Folie und Port herstellen. In vielen Fällen ist es üblich, dass der Beutel über Halterungen an den Einschweißports getragen oder hängend gelagert wird. Je nach Volumen eines jeweils befüllten Beutels übt die Folie an der Einschweißstelle über die Schwerkraft des Füllguts hohe Zugkräfte auf die Einschweißstelle aus. Für großvolumige Beutel wären die technischen Anforderungen die beschriebene Einschweißstelle in dichtender und tragfähiger Weise herzustellen zu hoch, um ein Beutelsystem in der Massenfertigung bei annehmbaren Herstellkosten zu fertigen.

Die Zugkräfte, die sich durch die Schwerkraft und evtl. den inneren hydrostatischen Druck eines gefüllten großvolumigen Beutels auf die Folienwandungen auswirken, können zu einer Ausdünnung des Folienmaterials führen. Die Folienwand gibt dabei durch plastische oder elastische Verformung den Zugkräften nach. Folien können entsprechend konzipiert sein, um trotz dieser Verformungen stabil zu sein; insbesondere wird auf die WO 2011/128185 verwiesen. Darin ist eine elastisch dehnbare Folie beschrieben, die für die Herstellung eines großvolumigen Beutels vorgesehen ist.

Bei der mechanischen Belastung von Schweißabschnitten an großvolumigen Beuteln tritt das Problem auf, dass sich die Folie aus ihrer Schweißverbindung mit den Schweißrippen der Einschweißschiff'schen durch Ausdünnung ablösen kann. Ein solcher Effekt kann insbesondere deshalb auftreten, weil das Folienmaterial über die gewölbten Schweißrippen des Einschweißschiffschens für die Verschweißung gedehnt werden muss. Durch Schwerkraft oder hydrostatischen Druck des gefüllten Beutels wirken zusätzlich foliendehnende Kräfte auf diese Schweißstelle ein.

Wünschenswert ist es weiterhin, dass solche Kunststoffteile funktionelle Ausgestaltungen aufweisen, wie z.B. die Aufnahmeabschnitte für Zugangsports zum Beutel oder eine Haltevorrichtung, an der der Beutel befestigt werden kann. Diese Gebrauchsanforderungen machen zudem die Verwendung von einem mechanisch stabilen, biegesteifen Material erforderlich.

EP 1 554 177 zeigt ein Verfahren zum dichtenden Einschweißen von Schlauchports zwischen die Folienwandungen eines Lösungsbeutels. Die Schrift nimmt dabei Bezug auf das maschinelle Aufgreifen der Folien, das Einlegen des Schlauchports und des Verschweißens. Auf die Problematik der Foliendehnung wird eingegangen, jedoch zeigt die Schrift kein Kunststoffteil mit einem biegesteifen Material.

EP 1 438 090 zeigt einen Beutel mit zwei Zugangsports, die jeweils in zwei getrennte innere Bereiche des Beutels münden. Der Beutel weist eine Halterung auf, die aus einfachen Aufnahmelöchern im Folienmaterial bestehen. Eine derartige Haltevorrichtung ist für großvolumige Beutel ungeeignet, da das Folienmaterial unter der Belastung des gefüllten Beutels einreißen kann.

EP 1 642 614 B1 zeigt einen Konzentratbeutel mit einem Kunststoffteil, das funktional für die Zugabe/Entnahme von Flüssigkeit vorbereitet ist und die Halterungsfunktion des Beutels übernimmt. Das Kunststoffteil ist dichtend über Schweißrippen mit den Folienwänden verschweißt. Der Beutel nimmt ein Konzentrat zur Herstellung einer gesättigten Lösung auf, mit der eine Dialysierflüssigkeit hergestellt werden kann. Insgesamt verfügt der Beutel nur über ein geringes Gewicht, auch im gefüllten Zustand, so dass die Problematik, Dichtfunktion und Halterungsfunktion der Einschweißzone des eingeschweißten Hartteils zu gewährleisten, nicht zum Tragen kommt.

US 4,386,634 zeigt einen großvolumigen Behälter, in dem Trockenkonzentrate für die Herstellung einer Dialyselösung vorgelegt sind. Durch Zuleiten von Wasser wird ein flüssiges Konzentrat vorbereitet. Die Ausführung der Verbindung zwischen Folienwandung und Hartteil in befestigender und dichtender Hinsicht wird dabei nicht im Detail ausgeführt.

Der Erfindung lag daher die Aufgabe zugrunde, einen Beutel zu schaffen, der im Bereich der Zuleitungen und Ableitungen mechanisch belastbar ist, wobei der Beutel gleichzeitig eine hohe Sicherheit für die abdichtende Lagerung des Füllgutes gewährleistet.

### Beschreibung der Erfindung

Die Aufgabe wird durch den Gegenstand von Anspruch 1, einem Beutel zur Aufnahme einer medizinischen Flüssigkeit, insbesondere einer gebrauchsfertigen und/oder gebrauchten Flüssigkeit, wie sie in der extrakorporalen Blutbehandlung oder der Nierentherapie vorkommt, und durch den Gegenstand von Anspruch 12, einem Verfahren zum Herstellen eines Beutels, gelöst. Vorteilhafte Ausführungsformen werden durch die Merkmale der Unteransprüche dargestellt. Insbesondere wird die Aufgabe gelöst durch ein biegesteifes Kunststoffteil, das separat von der Umgrenzungslinie mit den Folienwandungen verschweißt ist.

Demnach wird in einem Herstellungsverfahren eines Beutels ein Zu- oder Abgangsport mit den Folienwandungen in eine Umgrenzungslinie des herzustellenden Beutels abdichtend verbunden. In einem von diesem dichtenden Schweißbereich getrennten Bereich wird ein biegesteifes Kunststoffteil mit den Folienwandungen verschweißt, um eine mechanisch belastbare, auf die mechanischen Ansprüche des Beutels ausgerichtete Verbindungszone zu bilden.

Der erfindungsgemäße Beutel ist aus Folienwandabschnitten aufgebaut. Bevorzugt bestehen die Wandungen des Beutels vollständig aus Folienmaterialien. Ein Folienmaterial zeichnet sich im Sinne der vorliegenden erfindungsgemäßen Lehre dadurch aus, dass es sich um eine flächenförmige, flexible, biegbare und dünne Kunststoffmasse handelt. Auch solche flächenförmige Kunststoffmassen sind von dieser Begrifflichkeit umfasst, die in Schlauchform vorliegen, aber flächenförmig flachgelegt werden können. Schichtdicken der für den erfindungsgemäßen Gegenstand verwendeten Folien liegen im Bereich von 50 bis 500 µm, insbesondere 80 bis 300µm, je nach Ausgestaltung auch im Bereich von 100 bis 250 µm. Der Begriff Beutel umfasst im Sinne der erfindungsgemäßen Lehre kollabierbare Behälter, die im Leerzustand durch ihr Eigengewicht in einer flachgelegten Form vorliegen. Der Begriff umfasst auch Ausführungsformen, in denen Teile der Beutelwandungen aus einem biegesteifen Material angefertigt sind, solange im Gesamtcharakter der Beutel im Leerzustand zusammengelegt vorliegen kann.

Charakterisierend für eine erfindungsgemäße Ausführungsform ist, dass wenigstens zwei Folien entlang einer gemeinsamen Umgrenzungslinie ein inneres umschlossenes Volumen und somit einen Beutel bilden. Die Umgrenzungslinie kann eine umfängliche Schweißnaht sein, die zwei übereinandergelegte Folien verbindet. Die Umgrenzungslinie kann aber auch so verstanden werden, dass sie nur abschnittsweise aus Schweißnähten der Folien besteht und in anderen Abschnitten eine gefaltete Folie darstellt. Dies ist insbesondere bei Schlauchfolien der Fall. Solche Folien werden flachgelegt, so dass die beiden Folienschlauchhälften übereinanderliegen und seitlich mindestens eine Folienfalte bilden, die ebenfalls als Begrenzungslinie zu verstehen ist. In weiteren Bereichen müssen die losen Enden der Folien verschweißt werden, um einen geschlossenen Beutel zu erhalten. Zugangsports werden zwischen die Folien eingearbeitet. Z.B. werden Schlauchstücke zwischen zwei Folien eingelegt und in der gemeinsamen Umgrenzungslinie der wenigstens zwei Folien abdichtend eingeschweißt oder verklebt, wie dies in der EP 1 554 177 gezeigt ist. Alternativ können die Ports auch aus biegesteifem Material gebildet werden, die Schweißrippen beinhalten und in die gemeinsame Umgrenzungslinie der Folien abdichtend eingeschweißt werden.

Die Ports erfüllen die Funktion, einen Zugang zu dem inneren Beutelvolumen und dem äußeren Bereich herzustellen. Sie können mit weiteren Fluidführungsmitteln z.B. Schläuchen, Konnektoren und Adaptern verbunden sein, um eine Flüssigkeitszufuhr oder Flüssigkeitsentnahme zu gewährleisten.

Weiterhin zeichnet sich der erfindungsgemäße Beutel durch wenigstens ein in den Beutel eingearbeitetes biegesteifes Kunststoffteil aus, das ausgebildet ist, den wenigstens einen Zuführ-/Entnahmeport integral aufzunehmen. Unter biegesteif wird in diesem Zusammenhang verstanden, dass ein Gegenstand nicht ohne Zerstörung der funktionellen Ausrichtung geometrisch verformt werden kann.

Insbesondere werden unter biegesteifen Kunststoffen thermoplastische Kunststoffe verstanden, die bei einer Temperatur unterhalb ihrer Vicat Temperatur nach ISO 306 verwendet werden. Das biegesteife Kunststoffteil wird mit dem Beutel oder den Folienwandungen verbunden, worunter zu verstehen ist, dass das Kunststoffteil eingeschweißt, eingeklebt oder mit den Folien in einer Klemmwirkung verbunden wird. Weitere Befestigungsmethoden kommen äquivalent in Frage, sofern sie in der mechanischen Verbindung zu den Folienwandungen eine belastbare Verbindung ergeben. Insbesondere ist vorgesehen, dass der Beutel über das eingearbeitete Kunststoffteil mit einem Befestigungssystem oder einem Haltesystem verbunden werden kann. Dies setzt voraus, dass die Verbindung zwischen biegesteifem Kunststoffteil und Folienwandung des Beutels so stabil sein muss, dass der Beutel z.B. hängend oder teilweise hängend gelagert werden kann. Insbesondere hat es sich gezeigt, dass in die Umfassungslinie des Beutels eingearbeitete dichtende Portzugänge und das zur mechanischen Befestigung und/oder Halterung des Beutels zusätzlich eingearbeitete biegesteife Kunststoffteil vorteilhaft durch getrennte Verbindungsbereiche mit den Folienwandungen des Beutels verbunden werden. Damit kann gewährleistet werden, dass die dichtenden Bereiche der Umfassungslinie um den Zugangsport nur der mechanischen Beanspruchung durch den hydrostatischen Fülldruck ausgesetzt sind. Dagegen sind die dichtenden Bereiche nicht den mechanischen Belastungen durch Zugkräfte der Folie auf die dichtenden Bereiche ausgesetzt, die durch z.B. eine hängende Lagerung des Beutels entstehen. Vorteilhaft muss die Verbindung zwischen biegesteifem Kunststoffteil und Folienwandungen dann nicht derart exakt ausgearbeitet sein, dass sie eine Dichtfunktion erfüllt und eine solche Dichtigkeit auch noch bei hängender Lagerung und den auftretenden Zugkräften auf diese Verbindungsstellen erhalten bliebe.

In einer Ausführung sind der Zugangsport oder weitere Ports, z.B. Entlüftungsports dabei durch Schweißung/Klebungen in die Umfassungslinie eingearbeitet. Über diese Umfassungslinie hinaus überstehende Folienabschnitte können mit dem biegesteifen Kunststoffteil verschweißt, verklemmt oder verklebt werden, so dass Dichtbereich und Haltebereich an verschiedenen Zonen des Beutels angreifen.

Das biegesteife Kunststoffteil kann weitere funktionelle Einheiten umfassen. Es kann für die Aufnahme eines weiteren Zugangsports vorbereitet sein, um weitere Flüssigkeiten dem Beutelinneren zuzuleiten, oder eine Ent-/Belüftung des Beutels bei Flüssigkeitsentnahme oder Flüssigkeitsbefüllung zu ermöglichen. In diesem Fall können die Zugangsports mit z.B. Septen ausgestattet sein, um über Spritzen weitere Komponenten der gelagerten Dialysierflüssigkeit zuführen zu können. Es können weiterhin für eine sterile Be- und Entlüfung des Beutels Hydrophobfilter von den Zugangsports umfasst sein.

Als bevorzugte Methode zur Herstellung von Verbindungen zwischen Beutelfolie und den Ports empfehlen sich Schweißmethoden. Obwohl durch Klebungen evtl. ähnliche Verbindungseffekte der Beutelkomponenten erzielt werden könnten, sind produktionstechnisch die Schweißungen bevorzugt. Das Einschweißen der schlauchförmigen Zugangsports in die Umfassungslinie des Beutels wird ebenso bevorzugt durch Schweißverfahren durchgeführt. Unter Schweißverfahren sind z.B. auch Laserdurchstrahlschweißverfahren zu verstehen, wie sie z.B. in der WO 2007/115803 erwähnt sind. Ebenfalls kann für das Einschweißen der Ports auf die Techniken der EP 1 554 177 zurückgegriffen werden.

Die Trennung der dichtenden Einschweißung der Ports und der Einschweißung des funktionalen biegesteifen Kunststoffteils ist vor allem bei Beuteln mit einem großen Fassungsvolumen relevant. Insbesondere werden im Bereich der Dialyse mit Dialysierflüssigkeit gefüllte Beutel mit einem Volumen von 5 Litern angeboten, bei deren hängender Lagerung am Behandlungsort des Patienten durch das Eigengewicht bereits große mechanische Beanspruchungen an das Folienmaterial gestellt werden. Der erfindungsgemäße Gegenstand wird also insbesondere relevant für Beutelgrößen ab zwei Litern, wie sie z.B. in der Peritoneal Dialyse eingesetzt werden. Ebenfalls ist er relevant für Beutelgrößen von 5 Litern, die für Dialyse- oder Austauschflüssigkeiten in der Peritoneal Dialysse, der Hämodialyse oder der Hämofiltration Anwendung finden. In der Dialyse sind Konzepte bekannt, Beutelgrößen von bis zu 500 Litern für eine zentrale Versorgung mehrerer Dialysebehandlungstationen mit Dialysierflüssigkeit bereitzustellen. In der Akutdialyse können Beutelvolumina von bis zu 120 Litern vorgesehen sein, die in einem mobilen Tank gelagert werden, um mit einer Dialysebehandlungsstation verbunden zu werden. Insbesondere ist ein Beutel mit separater Schweißung der dichtenden Umfassungslinie und biegesteifem Kunststoffteil für Beutel der Größe von 5 bis 120 Litern relevant. Bevorzugt sind Beutelgrößen mit einem Fassungsvolumen von 30 bis 90 Litern, besonders bevorzugt 45 bis 75 Liter.

Insbesondere kommt der technische Vorteil einer getrennten Einschweißung von Ports in die dichtende Umfassungslinie und Aufnahme der Ports in das getrennt eingeschweißte biegesteife Kunststoffteil an einem Beutel zum Tragen, wenn der Beutel als Mehrkammerbeutel ausgebildet ist. In diesem Fall kann jeweils ein Port den Innenbereich einer Kammer mit dem äußeren Bereich des Beutels verbinden und so eine Mehrzahl von Ports durch das eingeschweißte biegesteife Kunststoffteil aufgenommen werden. Die Kammern können dabei unterschiedlich ausgebildet sein. In einer Ausführungsform wird eine Kammer aus einem inneren Beutel gebildet und ein den inneren Beutel umgebender Beutel bildet zwei weitere Kammern. Es besteht auch die Möglichkeit, dass die innere Kammer oder die äußeren Kammern weiter in Kompartimente unterteilt sind. Die Einteilung der Kompartimente erfolgt durch Begrenzungslinien, die bevorzugt aus Schweißnähten bestehen. In einer weiteren bevorzugten Ausführung bestehen die Begrenzungslinien wenigstens abschnittsweise aus Peelnähten.

Unter peelbaren Schweißverbindungen sind in diesem Zusammenhang Verbindungsstellen zweier Fügepartner zu verstehen, die durch Wärmebehandlung eine Adhäsivwirkung aufeinander ausüben. Im vorliegenden Fall handelt es sich bei den Fügepartnern um zwei gegenüberliegende Folienstücke eines Beutels, die im Schweißprozess mit einem Schweißbalken durch Wärmeeinwirkung und Anpressdruck miteinander verbunden werden. Die Schweißtemperatur bedingt, mit welcher Kraft die Peelnaht geöffnet werden kann. Unter einer Peelnaht ist eine Adhäsivverbindung zu verstehen, die durch Kraftwirkung wieder gelöst werden kann, ohne dass ein vollständiger Bruch des Folienmaterials erfolgt. In besonderen Ausführungen kann unter Peelverbindung auch eine solche Verbindung verstanden werden, die durch Krafteinwirkung eine teilweise Delamination eines mehrschichtigen Folienverbundes verursacht. In diesen Fällen ist es wichtig, dass der Delaminationsriss keinen vollständigen Bruch des Folienmaterials verursacht, was den Beutel unbrauchbar machen würde.

Das eingeschweißte biegesteife Kunststoffteil ist vorzugsweise dann so vorbereitet, dass es mehrere Ports aufnehmen kann, die jeweils die Verbindung zu mehreren Kammern oder Kompartimenten des Beutels herstellen. Es ergibt sich somit der Vorteil, dass eine Vielzahl der Ports an einem Kunststoffteil zusammengefasst wird. Dieser Vorteil macht sich insbesondere dann bemerkbar, wenn der Beutel über das Kunststoffteil in einer Halterung befestigt wird und die Ports mit weiteren Fluidleitungsmitteln, z.B. Schläuchen, Adaptern usw. verbunden werden sollen. Insbesondere kann in allen Ausführungsformen das biegesteife Kunststoffteil vorteilhaft weitere Funktionen integrieren, z.B. können Halterungen für weitere Gegenstände, z.B. Schläuche vorgesehen sein. Zudem können Abschnitte des Kunststoffteils von einer Schwächungslinie abgetrennt sein, so dass ein Abschnitt des Kunststoffteils abgebrochen werden kann, um anzuzeigen, dass der Beutel bereits verwendet wurde.

Es ist dabei für den Verbindungsbereich zwischen Folienwandungen und biegesteifem Kunststoffteil nicht notwendig, dass diese Verbindung dichtend ausgeführt wird. Produktionstechnisch vorteilhaft ist nur darauf zu achten, dass diese Verbindung so stabil ausgeführt ist, dass sie alle mechanischen Kräfte durch die Belastung des gefüllten Beutels aufnehmen kann. Insbesondere sind solche Kräfte gemeint, die bei einer Befestigung des Beutels über das biegesteife Kunststoffteil einwirken. Eine
Befestigung des Beutels kann dabei so aussehen, dass der Beutel frei hängend gelagert und über das eingeschweißte Kunststoffhartteil getragen wird. Dafür ist das Kunststoffhartteil mit Ösen oder einer Halteschiene ausgestattet, die im Eingriff mit komplementären Haltevorrichtungen stehen. Insbesondere bietet sich der Vorteil bei der Integrierung von Halteelementen in das biegesteife Kunststoffteil, diese in einer asymmetrischen Form auszugestalten. Die Halteelemente des Beutels und die komplementäre Haltevorrichtung zur Aufnahme des Beutels können so nur in einer bestimmten Beziehung in haltenden Eingriff gebracht werden. Damit wird sichergestellt, dass eine falsche Befestigung oder ein falscher Anschluss des Beutels in Verbindungsteile der Dialysebehandlungsstation unmöglich gemacht wird.

Bei sehr großen Beuteln ist zusätzlich zu der Halterung über das eingeschweißte biegesteife Kunststoffteil eine Stützapparatur vorgesehen, die den Beutel zumindest abschnittsweise umgibt und abstützt, um die Kräfte auf die Verbindung zwischen Folienwandungen und biegesteifem Einschweißsteil zu reduzieren. Vorteilhaft wird für den Beutel in einer großvolumigen Ausführung zwischen 5 Litern und 120 Litern ein Folienmaterial verwendet, das elastisch dehnbar ist. Bei Befüllung des Beutels vergrößert sich das Fassungsvolumen des Beutels mit zunehmender Befüllung ähnlich wie beim Aufblasen eines Luftballons. Die Verwendung solcher elastisch dehnbarer Folienmaterialien hat für großvolumige Beutel den Vorteil, dass mit weniger Folienmaterial ein großes Fassungsvolumen des gefüllten Beutels bereitgestellt werden kann. Es ist so mithin möglich, dass der Beutel im ungedehnten Zustand ein Volumen von 5 Litern aufweisen kann und im gefüllten Zustand ein Volumen von 60 Litern umfasst. Ein Folienmaterial, das bevorzugt zur Verwendung kommen kann, ist in der WO 2011/128185 beschrieben.

Der erfindungsgemäße Gegenstand wird bei der Ausgestaltung von Beuteln mit dehnbarem Folienmaterial besonders wichtig. Die Dehnung des Beutels verursacht eine Ausdünnung der Folien. Die Wahl solcher Folienmaterialien erfordert daher höhere Präzision in der Ausführung der dichtenden Schweißnaht. Insofern wird es besonders schwierig, eine Schweißnaht gleichzeitig dichtend und mechanisch belastbar auszugestalten. Erfindungsgemäß werden daher Schweißbereiche, die dichtende Funktion übernehmen, von Schweißbereichen, die die mechanische Halterung des Beutels übernehmen getrennt.

In einigen Ausführungen ist es möglich, den nicht dichtenden Schweißnahtbereich für weitere Funktionen zu verwenden, als die bereits erwähnte Aufnahme des biegesteifen Kunststoffteils. Insbesondere ist in einer Ausführung vorgesehen, einen Informationsträger durch Schweißung oder Verklebung oder Klemmung mit den Bereich zu verbinden, der die Verbindung zwischen biegesteifem Kunststoffteil und Folienabschnitten bildet. Der Informationsträger kann als bedrucktes Folienstück ausgebildet sein, der Informationen über den Beutel und des darin enthaltenen Produkts wiedergibt. Der Informationsträger kann maschinell auslesbare Informationen enthalten, die optisch oder magnetisch oder induktiv über ein Lesegerät verarbeitet werden können. Beispiele für optische Informationsübertragung sind maschinenlesbare Strichcodes oder Pixel codes, z.B. Dot Matrix Codes, sowie Farbcodierungen und ähnliches. Magnetische Übertragung kann durch Auslesen von Informationen auf Magnetstreifen erfolgen. Bekannt sind auch induktive Informationsübertragung nach RFID Technik, die ebenfalls in einer Ausführungsform auf dem Informationsträger mit dem Beutel verbunden sein können. In einer weiteren Ausgestaltung ist der Informationsträger über lösbare Mittel mit dem Beutel und/oder dem nicht dichtenden Bereich zwischen Folien und biegesteifem Kunststoffteil verbunden. Lösbare Mittel können z.B. perforierte Folienabschnitte sein, die ein Abtrennen des Inforniationsträgers ermöglichen. Alternativ ist der folienartige Informationsträger über eine Peelverbindung mit dem Beutel verbunden. In diesem Fall kann der Informationsträger auch separat von dem nicht dichtenden Verbindungsbereich angeordnet sein.

Der erfindungsgemäße Gegenstand umfasst ebenfalls ein Verfahren zum Herstellen eines Beutels, wobei dichtende Schweißbereiche und mechanisch tragfähige Schweißbereiche voneinander getrennt vorliegen. Bei dem erfindungsgemäßen Verfahren werden flache Folien parallel übereinander gelegt und entlang einer Umfassungslinie miteinander durch Schweißung oder Klebung verbunden. Bevorzugt werden Schweißverfahren verwendet. Wird der Beutel über Schlauchfolien aufgebaut, so wird ein flachgelegter Folienschlauch für die Herstellung des Beutels verwendet, so dass ein oberer Folienabschnitt parallel einem unteren Abschnitt gegenüberliegt. Für die Herstellung der Umfassungslinie muss der Folienschlauch nun seitlich nicht abgeschweißt oder verklebt werden. In diesem Fall bildet der im flachgelegten Zustand gebildete Folienknick einen Abschnitt der Umfassungslinie.

Darauf folgend werden die Portzugänge zwischen die zu verbindenden Folien positioniert, wobei vorzugsweise Schlauchports verwendet werden. Mit der Herstellung der verbindenden Umfassungslinie der beiden Folien werden die Schlauchports in der Umfassungslinie dichtend eingebaut. Vorzugsweise werden. Der innere Beutel weist um das Portende 107b eine runde Schweißverfahren, z.B. Ultraschallschweißen, Spiegelschweißen, Kontaktschweißen oder Laserdurchstrahlschweißen verwendet, um die dichtende Umfassungslinie mit den eingearbeiteten Ports herzustellen.

In einem weiteren Schritt wird das biegesteife Kunststoffteil mit Folienabschnitten des Beutels verbunden. Hierzu wird an dem Bereich, an dem die Ports in die Umfassungslinie eingearbeitet sind, ein überstehender Folienabschnitt genutzt. Das Kunststoffteil wird mit den überstehenden Folienabschnitten verbunden und zwar kann dies über Schweißtechniken, Klebetechniken oder Verklemmungen erfolgen. Bevorzugtes Verbindungsverfahren sind Schweißverfahren. Dabei kann das Kunststoffteil in unmittelbarer Nachbarschaft zu der Umfassungslinie liegen, in der die Ports dichtend umschlossen sind.

Das biegesteife Kunststoffteil kann dafür vorbereitet sein, den Einschweißport aufzunehmen. Z.B. können ein oder mehrere Ports in Hohldurchgänge des Kunststoffteils gesteckt werden. Die Verbindung zwischen Port und Aufnahme am biegesteifen Kunststoffteil kann durch Klebung, Wärmeverblockung oder Schweißung, insbesondere Laserdurchstrahlschweißung geschehen. Wärmeverblockung ist dabei ein Verkleben der Materialien von Schlauchport und biegesteifem Kunststoffteil durch Wärmeeinwirkung. Insbesondere ist unter der Aufnahme der Ports am biegesteifen Kunststoffteil auch zu verstehen, dass Port und biegesteifes Kunststoffteil ein integrales Bauteil bilden. Die Portabschnitte selbst können aus dem Material des biegesteifen Kunststoffteils bestehen.

In einer Ausführungsform kann der Port-Aufnahmebereich am biegesteifen Kunststoffteil ein Septum umfassen, um einen durchstechbaren Zuführport zu bilden. Ein weiterer Aufnahmebereich kann einen Filter beinhalten, insbesondere einen Hydrophobfilter, um den Beutel bei Befüllung oder Entnahme von Flüssigkeit keimarm zu entlüften. Der Filter ist dabei als Sterilfilter ausgebildet, der den Transport von Mikroorganismen oder Endotoxinen wirksam ausschließt.

Obwohl hiermit ein Ablauf von Herstellschritten beschrieben ist, der den erfindungsgemäßen Gegenstand beschreibt, so muss es als völlig äquivalent angesehen werden, die Abfolge der Herstellschritte in einer geänderten Reihenfolge ablaufen zu lassen. Insbesondere ist es nicht entscheidend, ob die Ports zuerst mit den Folienabschnitten des Beutels verbunden werden und anschließend mit den Aufnahmebereichen des biegesteifen Kunststoffteils oder umgekehrt. Im letzteren Fall werden die Schlauchports in einem ersten Schritt mit dem Aufnahmebereich am biegesteifen Kunststoffteil durch Klebung, Verblockung oder Verschweißung verbunden. In einem weiteren Schritt wird dieses zusammengestellte Teil mit dem Folienmaterial verschweißt. Dabei wird das zusammengestellte Teil mit den Portenden zwischen die übereinandergelegten Folien positioniert. Anschließend erfolgt der Produktionsschritt, der das biegesteife Kunststoffteil mit den Folien verbindet oder es wird zunächst die dichtende Umfassungslinie hergestellt, die den oder die Ports dichtend umschließt.

Erfindungsgemäß ist vorgesehen, dass die Verbindungsbereiche zwischen biegesteifem Kunststoffmaterial und den Folien nicht dichtend ausgeführt werden. Eine dichtende Verbindung, z.B. Schweißverbindung erfordert eine hohe Fertigungsgenauigkeit. Folienabschnitte müssen exakt gedehnt werden, um gewölbte Oberflächen einzuschweißender Hartteile oder Schlauchports dichtend zu umschließen. Aus diesem Grund reicht es, wenn die Schweißverbindung zwischen biegesteifem Kunststoffteil und Folie nur in solchen Abschnitten erfolgt, in denen eine schnelle, ohne Materialdehnung einhergehende Schweißung erfolgen kann. Bevorzugt werden daher nur ebene und parallel ausgerichtete Flächen von Folienwand und Kunststoffhartteil verschweißt. Die Aufnahmebereiche der Ports im Kunststoffteil werden bevorzugt von der Schweißung ausgenommen.

Selbstverständlich kann das erfindungsgemäße Verfahren auch für Mehrkammerbeutel angewendet werden, insbesondere auch für sogenannte "bag in bag" Konstruktionen, bei denen ein innerer Beutel ein erstes Fassungsvolumen definiert und ein äußerer, den inneren Beutel umgebender Beutel, ein weiteres zweites Fassungsvolumen und gegebenenfalls ein drittes Fassungsvolumen bildet. In diesem Fall geht man von vier übereinandergelegten Folien aus, wobei die untere Folie auch als eine Hälfte einer schlauchförmigen Folie verstanden werden kann. Unter dieser Begrifflichkeit ist auch zu verstehen, dass zwei ineinandergeführte und flachgelegte Schlauchfolien eine Abfolge von vier übereinandergelegten Folien ergeben. Ebenso würden eine flachgelegte Schlauchfolie und zwei Einzelfolien eine Abfolge von vier Folien ergeben. In einem weiteren Schritt werden die Ports zwischen die Folien positioniert und zwar so, dass sie bereits dem Kammerbereich des fertigen Beutels zugeordnet sind. Ein Port kann also zwischen erster und zweiter Folie oder zwischen zweiter und dritter Folie oder zwischen dritter und vierter Folie positioniert werden. Anschließend erfolgt wie zuvor beschrieben die Ausbildung der dichtenden Umfassungslinien mit den dichtend eingefassten Ports, so dass in diesem Bereich die vier Folien und der jeweilige Port von der gemeinsamen Umfassungslinie verbunden werden. Man erhält so einen Beutel mit mehreren Kammern und nach jeweiliger angestrebter Ausführungsform ist jede Kammer mit einem Portzugang versehen.

Mit der Ausbildung der Umfassungslinie oder der Verbindung zwischen Kunststoffteil und Folienwandungen kann ein weiteres funktionelles Folienstück verbunden werden. Es hat sich von Vorteil erwiesen, an diesen Stellen einen Informationsträger anzubringen, der in lesbarer Form oder in maschinenauslesbarer Form Informationen über das Produkt enthält. Insbesondere wenn ein Beutel mit elastisch dehnbarer Folie verwendet wird, können Bereiche des Beutels nicht mit lesbaren Informationen bedruckt werden, da bei Befüllung des Beutels und Dehnung der Folien das Druckbild zerstört würde. Es hat sich daher als notwendig erwiesen, den Informationsträger separat an den Beutel anzubringen, so dass die Foliendehnung keinen Einfluss nehmen kann. Insbesondere kann eine angebrachte Folie, die die Informationen enthält, z.B. in Schriftform, als Strichcode, als Dot Matrix Code, als Magnetstreifen oder RFID-Chip auf Vorder- und Rückseite mit Informationen versehen werden. Die Verbindung zur Umfassungslinie oder zum Verbindungsbereich zwischen Kunststoffteil und Folienwand kann peelbar sein oder aus einer Klebeverbindung bestehen, so dass der Informationsträger entfernt und z.B. in ein Behandlungsprotokoll, eine Patientenakte eingelegt werden kann.

### Zusammenfassung der Figuren

**Fig. 1** zeigt eine schematische Ansicht eines erfindungsgemäßen Beutels, insbesondere eines Mehrkammerbeutels, mit in der Umgrenzungslinie dichtend aufgenommenen Ports und einem davon separat eingearbeiteten biegesteifen Kunststoffteil.
**Fig. 2** zeigt eine weitere Ansicht in einer seitlichen Perspektive des Beutels.

### Detaillierte Beschreibung der Erfindung anhand der Zeichnungen

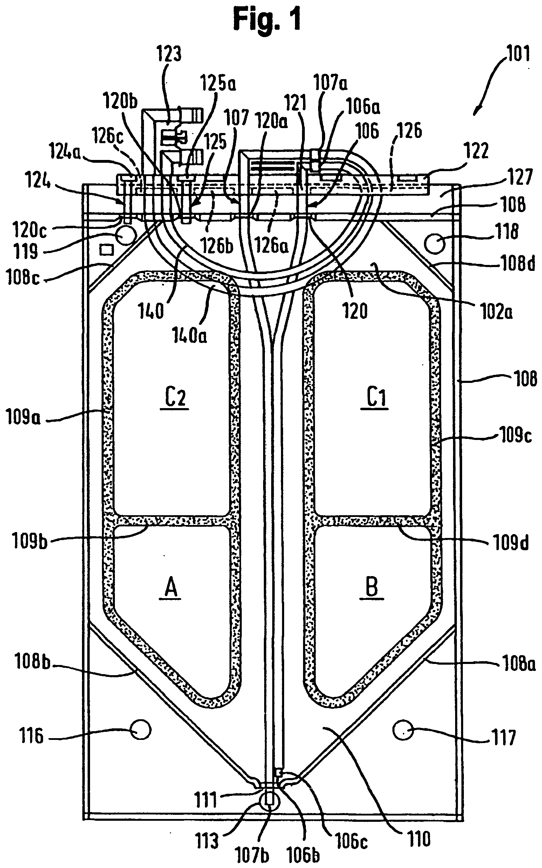

Der Beutel zeigt, wie in Fig. 1 schematisch dargestellt, eine obere Außenwandung 102a und eine untere, nicht gezeigte Außenwandung 102b auf, die durch einen gemeinsamen umfänglichen Rand 108 begrenzt und fluiddicht verbunden sind. In einer bevorzugten Version sind 102a und 102b die oberen und unteren Folien eines Beutels, die dichtende Umfassungslinien 108 wird durch eine gemeinsame permanente Schweißnaht gebildet.

Der Beutel verfügt über ein erstes Kompartiment A und ein weiteres Kompartiment C2, die mit Konzentraten für die Herstellung von Dialysierflüssigkeiten befüllt sind. Die Kompartimente A und C2 werden entlang einer geschlossenen Umfassungslinie von einer Trennlinie 109a umschlossen, die in der dargestellten Ausführungsform aus einer wenigstens abschnittsweise peelbaren Trennlinie besteht. Die Inhalte der Kompartimente A und C1 werden durch die weitere Trennlinie 109b, voneinander getrennt. Die weitere Trennlinie 109b kann aus einer permanenten Schweißlinie, einer teilweise semipermanenten Schweißlinie oder einer Peelnaht bestehen. Vorzugsweise sind die Trennlinien 109a und 109b als Peelnähte ausgestaltet und bilden eine zusammenhängende integrale Konstruktion von Peelnahtabschnitten.

Die beispielhafte Ausführung nach **Fig. 1** ist weiterhin dadurch gekennzeichnet, dass ein Verbindungsport 106 die Füllkammer 110 des Beutels über ein erstes Ende im Inneren des Beutels 106b und einem weiteren Ende 106a außerhalb des Beutels 101 mit dem Äußeren des Beutels verbindet. Port 106 ist vorzugsweise durch Schweißverbindungen fluiddicht in der dichtenden Umfassungslinie 108 an der Schweißzone 120 befestigt. Darüber hinaus wird Port 106 vom Aufnahmebereich 121 des biegesteifen Kunststoffteils 122 dichtend durch Schweißung, Klebung oder Verblockung aufgenommen. Vorzugsweise befindet sich am Ende 106b des Ports 106 ein Mittel 106c zur Erzeugung von Fluidturbulenzen des zuströmenden Verdünnungsmittels. Dieses Mittel kann als Turbulenz erzeugende Düse ausgestaltet sein oder als eine Turbulenz erzeugende Fritte. Weiterhin besteht Port 106 aus einem Schlauch, der den Beutel im Inneren in seiner länglichen Ausdehnung durchmisst. Es wird so im Befüllverfahren gewährleistet, dass bei aufrechter Lagerung des Beutels, zum Beispiel durch Aufnahme des Beutels an einer Halteschiene durch das biegesteifen Kunststoffteils 122, der Beutel von unten befüllt wird und die Kompartimente A, B, C1, C2 in der Abfolge A gleichzeitig mit B, vor C1 und C2 durch den inneren Fülldruck geöffnet werden.

Ein weiterer Port 107 mit einem ersten Ende 107a außerhalb des Beutels und einem weiteren Ende 107b dient zur Rückführung verbrauchter medizinischer Flüssigkeit, bevorzugt gebrauchte Dialyselösung, in eine weitere Kammer des Beutelsystems. Port 107 ist dabei im Inneren des Beutels als Schlauch ausgebildet und dafür vorgesehen, bei hängender Lagerung des Beutels, zum Beispiel durch Aufnahme des Beutels an einer Halteschiene des biegesteifen Kunststoffteils 122, den Beutel entlang einer länglichen Ausdehnung zu durchmessen. An der Schweißzone 111 durchdringt der Schlauch 107 die Beutel Umfassungslinie 108a des Beutels 101 und mündet in eine weitere, nicht gezeigte Kammer. 111 kann dabei eine fluiddichtende Schweißstelle sein, die den Beutel zwischen den oberen und unteren Begrenzungsebenen 102a und 102b fixiert und Teil der geschweißten Umfassungslinie 108 ist. Die nicht dargestellte Kammer ist ein umhüllender Container, bevorzugt Beutel, der integraler Bestandteil des Containersystems 101 ist. Es ergibt sich so eine "bag in bag" Konstruktion, bei der der Beutel, der die gebrauchsfertige Flüssigkeit aufnimmt, von einem Beutel, der die gebrauchte Flüssigkeit aufnimmt, umhüllt wird.

Weiterhin zeigt die Ausführung in **Fig. 1** einen zweiten Satz von Konzentratkammern B, C1, die durch eine weitere Trennlinie entlang einer geschlossenen Umfassungslinie 109c umschlossen sind. Eine weitere Trennlinie 109d trennt die Inhalte der Konzentratkammern B und C2. In einer bevorzugten Ausführungsform bilden die als Peelnähte ausgestalteten Linien 109c und 109d eine integrale Konstruktion aus Peelnahtabschnitten, die ineinander übergehen.

In der beispielhaften Ausführungsform enthält Kompartiment B ein Glukosekonzentrat in pulverförmiger und/oder wasserfreier oder in flüssiger Form. Eine weiteres Kompartiment C1 enthält ein Konzentrat, das mit dem Konzentrat der Kompartimente A und B unverträglich ist, also zur Degradation neigen oder unerwünschte Wechselwirkung eingehen würde.

Die Ausführungsform zeigt weiterhin eine Umfassungslinie 108, die vorzugsweise aus einer permanenten Schweißnaht besteht. Zusätzliche permanente Schweißlinien, Abschnitte 108a und 108b begrenzen den Containerinhalt oder Beutelinhalt, so dass ein schräger Boden des Innenraums entsteht. Die Konstruktion begünstigt die Verwirbelung des zuströmenden Verdünnungsmittels durch das Fluidturbulenz erzeugende Mittel 106c und damit den Lösungsprozess der Konzentrate aus den Kompartimenten A, B, C2, C1. Begrenzungslinien 108c und 108d verleihen dem gefüllten Container, insbesondere Beutel, weitere Stabilität im gefüllten Zustand. Dies ist insbesondere für großvolumige Container von Bedeutung, bei denen der Innendruck durch die beinhalteten Mengen belastende Spannungswirkung auf die Umfassungslinie 108 ausüben kann. Großvolumige Beutel sind in diesem Sinne als Container zu verstehen, die ein Volumen von 5 bis 120 Litern, 40 bis 80 Litern, insbesondere 60 Litern ± 15% aufweisen.

In einer Ausführung wird die Füllkammer über den Port 106 unter Lösen der Konzentrate aus den Kompartimenten A, B, C1, C2 gefüllt. Zur Entnahme der hergestellten Lösung wird über ein externes Pumpenmittel, wie z.B. eine Schlauchrollenpumpe oder eine Membranpumpe der Inhalt für die Dialysetherapie oder andere verarbeitende Prozesse entnommen. Nach Gebrauch der entnommenen Flüssigkeit wird diese über den Port 107 in das Beutelsystem zurückgeführtAussparung 113 der gegenüberliegenden Folien auf, so dass das Portende in Fluidverbindung mit dem Volumen des äußeren umhüllenden Beutels steht.

**Fig. 1** zeigt weiterhin, dass die Portenden 107a, 106a mit weiteren Fluidführungsmitteln, in diesem Fall die Schläuche 140, 140a, verbunden sind. Die Schläuche wiederum weisen an ihren Enden ein Konnektorteil 123 auf, das dafür vorgesehen ist, die Dialysierflüssigkeit in Fluidverbindung mit einer weiteren Flüssgikeitsverarbeitungsvorrichtung, z.B. einer Dialysemaschine zu bringen.

Mit 124 und 125 sind Belüftungsports bezeichnet. 125 kann beispielsweise mit der Füllkammer 110 des Beutels in Beziehung stehen, wobei Port 124 mit dem umhüllenden Beutel in Beziehung steht. Bei Befüllung der Kammer 110 des Beutels 101 über den Port 106 kann überschüssige Luft über den Entlüftungsport 125 entweichen. Bei Rückführung gebrauchter Flüssigkeit über den Port 107 in den nicht dargestellten umhüllenden Beutel kann eine notwendige Entlüftung über den Port 124 stattfinden. Die Entlüftungsports 124, 125, die die dichtende Umfassungslinie an den dichtenden Stellen 120c und 120b durchdringen, werden durch das biegesteife Kunststoffteil 122 aufgenommen. Das Kunststoffteil enthält dabei Aufnahmebereiche für Hydrophobmembranen 124a und 125a, so dass die Entlüftung der Kammern unter keimarmen Bedingungen erfolgen kann.

Insgesamt wird deutlich, das vier Ports 106, 107, 124, 125 dichtend die Umfassungslinie 108 an den Stellen 120, 120a, 120b, 120c durchdringen und von dem biegesteifen Kunststoffteil 122 aufgenommen werden. Das biegesteife Kunststoffteil selbst ist an den Schweißstellen 126, 126a, 126b, 126c mit der Folie 102a und der nicht gezeigten Folie 102b verschweißt. Wie zu sehen ist, sind die Schweißzonen nicht durchgängig.

Mit 116, 117, 118, 119 sind Durchgangsöffnungen im Folienmaterial des inneren Beutels bezeichnet, die bewirken sollen, dass bei Befüllung des nicht gezeigten umgebenden Beutels mit gebrauchter Flüssigkeit über Port 107 und Öffnung 113 der umgebende Beutel in gleichmäßiger Weise befüllt wird.

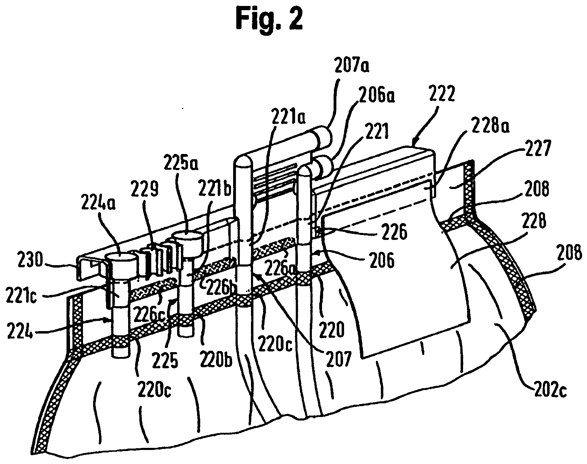

**Fig. 2** zeigt eine weitere Darstellung des erfindungsgemäßen Beutels. In dieser Darstellung ist der äußere Beutel gezeigt. Das Beutelsystem aus innerem und äußerem Beutel befindet sich in einem teilweise befüllten Zustand. Die Darstellung zeigt eine obere Folie 202c, die zusammen mit der nicht gezeigten rückwärtigen Folie 202d und der Verbindung entlang der Umfassungslinie 208 ein abgeschlossenes Fassungsvolumen des umgebenden Beutels definieren. Die Zugänge zum Inneren des äußeren Beutels und zum Inneren des nicht gezeigten inneren Beutels werden über die Ports 206, 207, 225, 224 hergestellt. Dabei stellen die Ports 206, 207 Fluidports zur Befüllung und Entnahme von Flüssigkeit dar. Die Ports 225 und 224 sind Zugänge zur keimarmen Be- und Entlüftung während Befüll- und Entleerungsvorgängen. Port 206 liefert dabei den Zugang zum inneren Beutel, Port 207 liefert den Zugang zum den inneren Beutel umgebenden äußeren Beutel. Analog stellt 225 einen Belüftungsport für den inneren Beutel dar und 224 ist ein Port zur Belüftung des äußeren Beutels. Die Ports sind an den Stellen 220, 220a, 220b, 220c, in die Umfassungslinie 208 eingearbeitet und an den Stellen 221, 221a, 221b, 221c von dem biegesteifen Kunststoffteil aufgenommen. An den Stellen 226, 226a, 226b, 226c ist das biegesteife Kunststoffteil mit dem die Umfassungslinie 108 überragenden Folienabschnitt 227 durch z.B. Verschweißung verbunden. Die Portaufnahmebereiche 221, 221 a, 221 b, 221 c sind von der Schweißung ausgenommen.

**Fig. 2** stellen 229 Schlauchclips zur Aufnahme der nicht gezeigten Schläuche, die an den Portenden 207a, 206a anschließen da. Die entsprechenden Schläuche werden in **Fig. 1** mit 140, 140a bezeichnet. Bevorzugt wird das Kunststoffteil 222 in einem Spritzgussverfahren hergestellt. Es ist daher möglich, eine Vielzahl von funktionellen Einheiten in einem Arbeitsprozess des Spritzgusses an dem Kunststoffteil zu integrieren. Insbesondere wird dadurch auch eine im Querschnitt durch die Längsausdehnung des Kunststoffteils U-förmige Form des Kunststoffteils hergestellt. In dieser Ausführung stellt das Kunststoffteil eine U-förmige Halteschiene dar, wie durch 230 angedeutet ist. Die Halteschiene kann nun an einer formkomplementären Haltevorrichtung angebracht werden und den Beutel in einer Aufnahmeeinheit einer Dialysebehandlungsstation befestigt werden. In Verbindung mit den weiteren funktionellen Einheiten des Kunststoffteils, z.B. Ports und Schlauchclips, kann bei entsprechender Anordnung eine asymmetrische Ausgestaltung des Kunststoffteils erreicht werden. Asymmetrisch meint in diesem Sinne, dass ein Spiegelbild des Kunststoffteils durch Drehung und Verschiebung nicht deckungsgleich auf dem Ursprungsbild abgebildet werden kann. Es kann, so wie in **Fig. 2** gezeigt, eine geometrische Codierung des Kunststoffteils 222 hergestellt werden, mit der der Beutel und das Kunststoffteil nur in eindeutiger Weise mit einer hier nicht gezeigten Haltevorrichtung befestigt werden kann. Dies ist insbesondere wichtig, um fehlerhafte Bedienung des Beutels zu vermeiden.

Mit 228 ist in **Fig.** 2 schematisch ein Informationsträger gezeigt. Dieser Informationsträger besteht aus einem folienartigen Material und ist über die Verbindungszone 228a mit der Schweißzone 226 nur einseitig verbunden. Das Folienmaterial kann bedruckt sein und durch die nur einseitige Befestigung umgeschlagen werden. Die Verbindungszone 228a kann lösbar sein, z.B. wenn für 228a Klebe- oder Verblockung- oder Peelnahtverbindungen gewählt werden.

## Patentansprüche

1. Beutel (101) für die Aufnahme von medizinischen Flüssigkeiten für die Nierentherapie, bestehend aus Folienwandabschnitten (102a, 102b, 202c, 202d), die umfänglich entlang einer Umgrenzungslinie (108, 208) wenigstens abschnittsweise verschweißt sind, mit wenigstens einem Zugangsport (106, 107, 206, 207), dessen erstes Ende (106a, 107a) einen inneren Bereich des Beutels mit dem Außenbereich in Fluidverbindung bringt und der abdichtend in die Umgrenzungslinie (108, 208) eingearbeitet ist und einem biegesteifen Kunststoffteil (122, 222), wobei das biegesteife Kunststoffteil (122, 222) separat von der Umgrenzungslinie (108,208) mit den Folienwandungen (102a, 102b, 202c, 202d) verbunden ist, **dadurch gekennzeichnet, dass** das biegesteife Kunststoffteil (122, 222) separat von der abdichtenden Umgrenzungslinie (108, 208) nicht abdichtend mit den Folienwandungen (102a, 102b, 202c, 202d) verschweißt ist, und dass im biegesteifen Kunststoffteil (122, 222) Aufnahmen für Zugang-/Entnahmeports (106, 107, 206, 207) und/oder Belüftungsports (124, 125, 224, 225) und/oder Schlauchhalterungen (229) integriert sind.

2. Beutel (101) nach Anspruch 1, **dadurch gekennzeichnet, dass** das biegesteife Kunststoffteil (122, 222) Aufnahmebereiche aufweist, die dafür vorbereitet sind, die Ports (106, 107, 124, 125, 206, 207, 224, 225) aufzunehmen.

3. Beutel (101) nach einem der Ansprüche 1 oder 2, **dadurch gekennzeichnet, dass** das biegesteife Kunststoffteil (122, 222) integrale Befestigungsmittel, insbesondere eine Halteschiene (230) und/oder einen oder mehrere Belüftungsports (124, 125, 224, 225) umfasst.

4. Beutel (101) nach Anspruch 3, **dadurch gekennzeichnet, dass** das biegesteife Kunststoffteil (122, 222) mit den integralen Befestigungsmitteln, insbesondere einer U-förmigen Halteschiene (230), eine asymmetrische geometrische Form ausbilden, so dass der Beutel (101) nur in eindeutiger Weise mit einem komplementären Haltesystem verbunden werden kann.

5. Beutel (101) nach einem oder mehreren der Ansprüche 1 bis 4, **dadurch gekennzeichnet, dass** der eine Zugangsport (106, 107, 206, 207) in die Umgrenzungslinie (108, 208) eingeschweißt ist.

6. Beutel (101) nach einem oder mehreren der Ansprüche 1 bis 5, **dadurch gekennzeichnet, dass** es sich um einen großvolumigen Beutel (101) handelt mit Fassungsvolumen von 5 bis 120 Litern, vorzugsweise 30 bis 90 Litern, vorzugsweise 45 bis 75 Litern.

7. Beutel (101) nach einem oder mehreren der Ansprüche 1 bis 6, **dadurch gekennzeichnet, dass** es sich um einen Mehrkammerbeutel (101) handelt.

8. Beutel (101) nach Anspruch 7, **dadurch gekennzeichnet, dass** mindestens zwei Kammern des Beutels (101) mit jeweils einem Zugangsport (106, 107, 206, 207) mit dem Außenbereich des Beutels (101) verbunden sind, die abdichtend die Umgrenzungslinie (108, 208) durchdringen.

9. Beutel (101) nach Anspruch 7 oder 8, **dadurch gekennzeichnet, dass** die jeweils zwei Zugangsports (106, 107, 206, 207) von dem biegesteifen Kunststoffteil (122, 222) aufgenommen werden.

10. Beutel (101) nach einem oder mehreren der Ansprüche 1 bis 9, **dadurch gekennzeichnet, dass** die Folienwandungen (102a, 102b, 202c, 202d) aus einem elastisch dehnbaren Material hergestellt sind.

11. Beutel nach einem oder mehreren der Ansprüche 1 bis 10, **dadurch gekennzeichnet, dass** im Schweißbereich des biegesteifen Kunststoffteils (122, 222) und der Folienwandung (102a, 102b, 202c, 202d) ein Informationsträger (228) befestigt ist.

12. Verfahren zur Herstellung eines Beutels (101), umfassend die Schritte
• Übereinanderlegen von mindestens zwei Folien oder Flachlegen eines Folienschlauchs
• Positionieren von mindestens einem Zugangsport (106, 107, 206, 207) zwischen die Folien oder zwischen gegenüberliegende Folienabschnitte (102a, 102b, 202a, 202b) des flachgelegten Folienschlauchs, so dass der Port (106, 107, 206, 207) von gegenüberliegenden Seiten von Folien oder Folienabschnitten (102a, 102b, 202c, 202d) benachbart ist
• Herstellen einer dichtenden Begrenzungslinie (108, 208) durch Schweißen und/oder Kleben der Folien oder des Folienschlauchs mit dem Port (106, 107, 206, 207)
• Positionieren eines biegesteifen Kunststoffteils (122, 222) in einem Bereich zwischen den über die dichtende Umfassungslinie (108, 208) hinausragenden Teil der Folien oder der Folienabschnitte (102a, 102b, 202c, 202d) des Folienschlauchs, wobei im biegesteifen Kunststoffteil (122, 222) Aufnahmen für Zugang-/Entnahmeports (106, 107, 206, 207) und/oder Belüftungsports (124, 125, 224, 225) und/oder Schlauchhalterungen (229) integriert sind
• Verbinden des Kunststoffteils (122) mit den überragenden Folienabschnitten (102a, 102b, 202c, 202d), wobei die Verbindung zwischen Folien oder Folienabschnitten (102a, 102b, 202c, 202d) und beigesteifem Kunststoffteil (122, 222) nicht dichtend ausgeführt ist.

13. Verfahren nach Anspruch 12, **dadurch gekennzeichnet, dass** der Port (106, 107, 124, 125, 206, 207, 224, 225) in einem Aufnahmebereich am biegesteifen Kunststoffteil (122, 222) befestigt wird.

14. Verfahren nach Anspruch 12 oder 13, **dadurch gekennzeichnet, dass** das biegesteife Kunststoffteil (122, 222) über Schweißung oder Klebung oder Klemmung mit den Folienabschnitten (102a, 102b, 202c, 202d) verbunden wird.

15. Verfahren nach einem oder mehreren der Ansprüche 12 bis 14, **dadurch gekennzeichnet, dass** vier Folien übereinandergelegt und entlang einer gemeinsamen Umfassungslinie (108, 208) verschweißt werden, wobei mindestens zwei gegenüberliegende Folien aus Folienabschnitten (102a, 102b, 202c, 202d) eines flachgelegten Folienschlauchs gebildet werden können, um einen Mehrkammerbeutel (101) mit einem inneren Beutel, umfassend eine innere Kammer, und einen den inneren Beutel umgebenden Beutel, umfassend eine zweite und/oder eine dritte Kammer, zu erhalten.

16. Verfahren nach einem oder mehreren der Ansprüche 12 bis 15, **dadurch gekennzeichnet, dass** ein weiteres Folienstück (228) mit der Verbindungstelle zwischen Folien oder Folienabschnitten (102a, 102b, 202c, 202d) und biegesteifem Kunststoffteil (122, 222) verbunden wird.

## Claims

1. A bag (101) for holding medical fluids for renal therapy consisting of film wall sections (102a, 102b, 202c, 202d), which are welded together peripherally along a bordering line (108, 208) in at least some sections, having at least one access port (106, 107, 206, 207) whose first end (106a, 107a) brings an inner area of the bag in fluid connection with the outer area and which is incorporated into the bordering line (108, 208) with a seal, and a flexurally rigid plastic part (122, 222), wherein the flexurally rigid plastic part (122, 222) is connected to the film walls (102a, 102b, 202c, 202d) separately from the bordering line (108, 208), **characterized in that** the flexurally rigid plastic part (122, 222) is welded without a seal to the film walls (102a, 102b, 202c, 202d) separately from the sealing bordering line (108, 208); and **in that** receptacles for access ports/withdrawal ports (106, 107, 206, 207) and/or vent ports (124, 125, 224, 225) and/or tube holders (229) are integrated into the flexurally rigid plastic part (122, 222).

2. The bag (101) according to Claim 1, **characterized in that** the flexurally rigid plastic part (122, 222) has receptacle areas which are prepared for receiving the ports (106, 107, 124, 125, 206, 207, 224, 225).

3. The bag (101) according to any one of Claims 1 or 2, **characterized in that** the flexurally rigid plastic part (122, 222) comprises integral fastening means, in particular a retaining rail (230) and/or one or more vent ports (124, 125, 224, 225).

4. The bag (101) according to Claim 3, **characterized in that** the flexurally rigid plastic part (122, 222) is able to form an asymmetrical geometric shape with the integral fastening means, in particular a U-shaped retaining rail (230), so that the bag (101) can be connected to a complementary restraint system only in an unambiguous way.

5. The bag (101) according to any one or more of Claims 1 to 4, **characterized in that** the one access port (106, 107, 206, 207) is welded into the bordering line (108, 208).

6. The bag (101) according to any one or more of Claims 1 to 5, **characterized in that** it is a large volume bag (101) with a capacity of 5 to 120 liters, preferably 30 to 90 liters, preferably 45 to 75 liters.

7. The bag (101) according to any one or more of Claims 1 through 6, **characterized in that** it is a multichamber bag (101).

8. The bag (101) according to Claim 7, **characterized in that** at least two chambers of the bag (101) are connected to the outer area of the bag (101) each by an access port (106, 107, 206, 207) penetrating through the bordering line (108, 208) with a seal.

9. The bag (101) according to Claim 7 or 8, **characterized in that** the two access ports (106, 107, 206, 207) are each accommodated by the flexurally rigid plastic part (122, 222).

10. The bag (101) according to any one or more of Claims 1 to 9, **characterized in that** the film walls (102a, 102b, 202c, 202d) are made of an elastically extensible material.

11. The bag according to any one or more of Claims 1 to 10, **characterized in that** an information carrier (228) is fastened in the welded area of the flexurally rigid plastic part (122, 222) and the film wall (102a, 102b, 202c, 202d).

12. A method for manufacturing a bag (101), comprising the steps
• placing at least two films one above the other or flattening a film tube,
• positioning at least one access port (106, 107, 206, 207) between the films or between opposing film sections (102a, 102b, 202a, 202b) of the flattened film tube so that the port (106, 107, 206, 207) is in proximity to opposing sides of films or film sections (102a, 102b, 202c, 202d),
• producing a sealing bordering line (108, 208) by welding and/or gluing the films or the film tube to the port (106, 107, 206, 207),
• positioning a flexurally rigid plastic part (122, 222) in an area between the part of the films or the film sections (102a, 102b, 202c, 202d) of the film tube protruding beyond the sealing bordering line (108, 208), wherein receptacles for access ports/withdrawal ports (106, 107, 206, 207) and/or vent ports (124, 125, 224, 225) and/or tube holders (229) are integrated into the flexurally rigid plastic part (122, 222),
• connecting the plastic part (122) to the protruding film sections (102a, 102b, 202c, 202d), wherein the connection between films or film sections (102a, 102b, 202c, 202d) and the flexurally rigid plastic part (122, 222) is not embodied to form a seal.

13. The method according to Claim 12, **characterized in that** the port (106, 107, 124, 125, 206, 207, 224, 225) is attached to the flexurally rigid plastic part (122, 222) in a receiving area.

14. The method according to Claim 12 or 13, **characterized in that** the flexurally rigid plastic part (122, 222) is connected to the film sections (102a, 102b, 202c, 202d) by welding or gluing or clamping.

15. The method according to any one or more of Claims 12 to 14, **characterized in that** four films are placed one above the other and are welded along a joint bordering line (108, 208), wherein at least two opposing films may be formed from film sections (102a, 102b, 202c, 202d) of a flattened film tube to obtain a multichamber bag (101) with an inner bag comprising an inner chamber and a bag surrounding the inner bag, comprising a second chamber and/or a third chamber.

16. The method according to any one or more of Claims 12 to 15, **characterized in that** another film piece (228) is connected to the connecting line between the films or film sections (102a, 102b, 202c, 202d) and the flexurally rigid plastic part (122, 222).

## Revendications

1. Poche (101) destinée à recevoir des liquides médicaux pour la thérapie rénale, constituée de sections de paroi en film (102a, 102b, 202c, 202d), qui sont soudées sur la périphérie le long d'une ligne de délimitation (108, 208) au moins sur certaines parties, comprenant au moins un orifice d'entrée (106, 107, 206, 207), dont la première extrémité (106a, 107a) met une zone intérieure de la poche en communication fluidique avec l'extérieur et qui est incorporé de manière hermétique dans la ligne de délimitation (108, 208) et une pièce en plastique rigide en flexion (122, 222), la pièce en plastique rigide en flexion (122, 222) étant reliée aux parois en film (102a, 102b, 202c, 202d) séparément de la ligne de délimitation (108, 208), **caractérisée en ce que** la pièce en plastique rigide en flexion (122, 222) est soudée aux parois en film (102a, 102b, 202c, 202d) de manière non hermétique séparément de la ligne de délimitation (108, 208) hermétique, et **en ce que** des logements sont intégrés dans la pièce en plastique rigide en flexion (122, 222) pour des orifices d'entrée/de prélèvement (106, 107, 206, 207) et/ou des orifices de sortie d'air (124, 125, 224, 225) et/ou des supports de tuyaux (229).

2. Poche (101) selon la revendication 1, **caractérisée en ce que** la pièce en plastique rigide en flexion (122, 222) comporte des zones de réception, qui sont préparées pour recevoir les orifices (106, 107, 124, 125, 206, 207, 224, 225).

3. Poche (101) selon l'une des revendications 1 ou 2, **caractérisée en ce que** la pièce en plastique rigide en flexion (122, 222) comprend des moyens de fixation intégrés, en particulier un rail de retenue (230) et/ou un ou plusieurs orifices de sortie d'air (124, 125, 224, 225).

4. Poche (101) selon la revendication 3, **caractérisée en ce que** la pièce en plastique rigide en flexion (122, 222) avec les moyens de fixation intégrés, en particulier un rail de retenue (230) en forme de U, présente une forme géométrique asymétrique, de sorte que la poche (101) peut être reliée à un système de retenue complémentaire uniquement de manière évidente.

5. Poche (101) selon une ou plusieurs des revendications 1 à 4, **caractérisée en ce que** l'orifice d'entrée (106, 107, 206, 207) est soudé dans la ligne de délimitation (108, 208).

6. Poche (101) selon une ou plusieurs des revendications 1 à 5, **caractérisée en ce qu'**il s'agit d'une poche (101) à grand volume, avec une capacité de 5 à 120 litres, de préférence 30 à 90 litres, de préférence 45 à 75 litres.

7. Poche (101) selon une ou plusieurs de revendications 1 à 6, **caractérisée en ce qu'**il s'agit d'une poche à compartiments multiples (101).

8. Poche (101) selon la revendication 7, **caractérisée en ce qu'**au moins deux compartiments de la poche (101) sont en liaison avec l'extérieur de la poche (101) au moyen respectivement d'un orifice d'accès (106, 107, 206, 207), qui pénètre dans la ligne de délimitation (108, 208) de manière hermétique.

9. Poche (101) selon la revendication 7 ou 8, **caractérisée en ce que** les respectivement deux orifices d'accès (106, 107, 206, 207) sont reçus dans la pièce en plastique rigide en flexion (122, 222).

10. Poche (101) selon une ou plusieurs des revendications 1 à 9, **caractérisée en ce que** les parois en film (102a, 102b, 202c, 202d) sont fabriquées en un matériau extensible de manière élastique.

11. Poche selon une ou plusieurs des revendications 1 à 10, **caractérisée en ce qu'**un support d'information (228) est fixé dans la zone de soudure de la pièce en plastique rigide en flexion (122, 222) et de la paroi en film (102a, 102b, 202c, 202d).

12. Procédé de fabrication d'une poche (101), comprenant les étapes consistant à
• superposer au moins deux films ou poser à plat un tube en film
• positionner au moins un orifice d'accès (106, 107, 206, 207) entre les films ou entre des sections de film (102a, 102b, 202a, 202b) opposées du tube en film posé à plat, de telle sorte que l'orifice (106, 107, 206, 207) est contigu à des côtés opposés de films ou de sections de film (102a, 102b, 202c, 202d)
• réaliser une ligne de délimitation (108, 208) hermétique par soudage et/ou collage des films ou du tube en film avec l'orifice (106, 107, 206, 207)
• positionner une pièce en plastique rigide en flexion (122, 222) dans une zone entre la partie des films ou des sections de film (102a, 102b, 202c, 202d) du tube en film qui dépassent de la ligne de contour (108, 208) hermétique, des logements étant intégrés dans la pièce en plastique rigide en flexion (122, 222) pour des orifices d'entrée / de prélèvement (106, 107, 206, 207) et/ou des orifices de sortie d'air (124, 125, 224, 225) et/ou des supports de tuyau (229)
• relier la pièce en plastique (122) aux sections de film (102a, 102b, 202c, 202d) dépassant, la liaison entre les films ou sections de film (102a, 102b, 202c, 202d) et la pièce en plastique rigide en flexion (122, 222) étant réalisée de manière non hermétique.

13. Procédé selon la revendication 12, **caractérisé en ce que** l'orifice (106, 107, 124, 125, 206, 207, 224, 225) est fixé à la pièce en plastique rigide en flexion (122, 222) dans une zone de réception.

14. Procédé selon la revendication 12 ou 13, **caractérisé en ce que** la pièce en plastique rigide en flexion (122, 222) est reliée aux sections de film (102a, 102b, 202c, 202d) par soudage ou collage ou serrage.

15. Procédé selon une ou plusieurs des revendications 12 à 14, **caractérisé en ce que** quatre films sont superposés et soudés le long d'une ligne de contour (108, 208) commune, au moins deux films opposés pouvant être formés à partir de sections de film (102a, 102b, 202c, 202d) d'un tube en film posé à plat, afin d'obtenir une poche à compartiments multiples (101) avec une poche intérieure, comprenant un compartiment intérieur, et une poche entourant la poche intérieure, comprenant un deuxième et/ou un troisième compartiment.

16. Procédé selon une ou plusieurs des revendications 12 à 15, **caractérisé en ce qu'**un autre morceau de film (228) est relié au point de liaison entre les films ou les sections de film (102a, 102b, 202c, 202d) et la pièce en plastique rigide en flexion (122, 222).
